Europäisches Patentamt

European Patent Office

Office européen des brevets

(11) Publication number: **0 144 486 B1**

# EUROPEAN PATENT SPECIFICATION

(45) Date of publication of patent specification: **27.11.91**

(51) Int. Cl.⁵: **A61F 13/00, A61K 9/24, A61L 15/16**

(21) Application number: **84103695.7**

(22) Date of filing: **04.04.84**

(54) **Controlled-release drugsystem and process for preparing it.**

(30) Priority: **27.04.83 DE 3315245**
     **27.04.83 DE 3315272**

(43) Date of publication of application:
    **19.06.85 Bulletin 85/25**

(45) Publication of the grant of the patent:
    **27.11.91 Bulletin 91/48**

(84) Designated Contracting States:
    **AT BE CH DE FR GB IT LI LU NL SE**

(56) References cited:
    **EP-A- 0 020 905**
    **EP-A- 0 050 480**
    **CH-A- 574 742**

    **CHEMICAL ABSTRACTS, vol. 96, no. 22, May
    1982, page 420, no. 187302x, Columbus,
    Ohio, US.**

(73) Proprietor: **Lohmann GmbH & Co. KG
    Irlicher Strasse 55
    W-5450 Neuwied 12(DE)**

Proprietor: **Schwarz Pharma Aktiengesell-
    schaft**
    **Mittelstrasse 11-13
    W-4019 Monheim(DE)**

(72) Inventor: **Hoffmann, Hans Rainer, Dr.
    Burghofstrasse 113
    W-5450 Neuwied 22(DE)**
    Inventor: **Meconi, Reinhold
    Alemannenstrasse 42
    W-5450 Neuwied 11(DE)**
    Inventor: **Wolff, Michael, Dr.
    Richard-Wagner-Strasse 2
    W-4019 Monheim(DE)**
    Inventor: **Zerbe, Horst, Dr.
    Johann-Sebastian-Bach-Strasse 82
    W-4019 Monheim(DE)**

(74) Representative: **Redies, Bernd, Dr. rer. nat. et
    al
    COHAUSZ & FLORACK Patentanwaltsbüro
    Schumannstrasse 97 Postfach 14 01 20
    W-4000 Düsseldorf 1(DE)**

## Description

The present invention is related to a pharmaceutical product for the administration of therapeutically active agents to and through the skin in the form of a medical bandage.

The administration of therapeutically active agents to the skin for instance of human beings from medical bandage-like products is known. It is the purpose of this kind of administration to obtain in particular in transdermal administration a release of active agent as uniform as possible over a prolonged period of time and to obtain thereby an uptake of the active agent through this skin as uniform as possible. The control of active agent release from the medical bandage into the skin has at first been achieved by providing a particular membrane on the reservoir for the active agent towards the skin controlling the drug release rate (see for instance German patent 2,135,533; US patents 3,598,122 and 3,797,494). The release of active agent occurs by membrane controlled diffusion. The role of the controlling membrane may also be achieved by a pressure-sensitive adhesive coating, as described in European patent publication 33615. In this way it is possible to avoid the relatively expensive and complicated controlling membrane. However, in order to obtain medical bandages in a size necessary for the intended long-term treatment and acceptable for the patient, limitations in the possible dosages per medical bandage unit cannot be avoided.

In German patent publication DE-OS 3119752 a similar system is described. However, the speed of dissolution of the active agent in the reservoir layer is controlling for the release rate of the active agent from the system. Furthermore, the practical use of this system however shows that with this embodiment of medical bandage there is a limitation of the amount of active agent releasable per bandage unit and there is no uniformity of active agent release.

Furthermore, in German patent publication DE-OS 292o5oo a transdermal system is described where in a swellable polymer foil with a single reservoir layer the concentration of active agent decreases from the release surface in order to get a uniform release rate. It has been proposed furthermore in this prior art to combine several films of this type. However, the same active agent concentration profile is present in each layer. The particular active agent concentration profile in this unilayer film is produced by diffusion of a solution or suspension of the active agent from one surface of the film into the film and removal of the solvent or, respectively, suspension agent. This process has the considerable disadvantage that the desired active agent concentration profile in the film may reproduced only with considerable technical difficulty.

It is a further disadvantage in this process that the active agent uptake of the film is limited by its adsorption capability. Furthermore, the combination of several such films yields in so complicated conditions that a uniform release of active agent can no more be reached. It is therefore an object of the present invention to avoid these disadvantages and to provide a pharmaceutical product, preferably in medical bandage form, which may be produced in a simple and cheap manner and which allows a reproducible controlled active agent release over the complete time of use and which also allows a change in the release rate.

The pharmaceutical product according to the present invention in medical bandage form, for controlled release of a therapeutically active agent to the skin having a reservoir layer of a polymer material permeable to the therapeutically active agent, wherein the therapeutically active agent is soluble and wherein there is a profile of concentration of the therapeutically active agent such that the concentration is higher in the area farther from the surface of the reservoir towards the skin than closer thereto and having an adhesive layer on the side towards the skin, is characterised in that the medical bandage consists of an impermeable backing layer, the reservoir layer adjacent to, and in close contact with, said backing layer, the adhesive layer adjacent to, and in close contact with, said reservoir layer and a cover layer covering and adhering to said adhesive layer and removable therefrom for the use of said pharmaceutical product as transdermal therapeutic system, said reservoir layer for the therapeutically active agent

a) consisting of a multitude of individual polymer matrix layers,

b) each said individual layer having a concentration of said therapeutically active agent above saturation with the therapeutical active agent both in dissolved and undissolved state, and

c) the concentration of the therapeutically active agent in said individual layers increasing from individual layer to individual layer with increasing distance from said adhesive layer.

In the attached drawings

Figure 1    shows a cross section of a pharmaceutical product according to the present invention with a two-layer reservoir (the shown layer thicknesses are not corresponding to the actual thickness);

Figure 2    shows a cross section of a different embodiment with a five-layer reservoir (the shown layer thicknesses do not correspond to the actual thickness);

Figure 3    shows a graphical diagram of the release of nitroglycerin from a medical bandage according to the present invention over the time;

Figure 4    shows the plasma concentration of nitroglycerin as obtained with a medical bandage according to the present invention over the time.

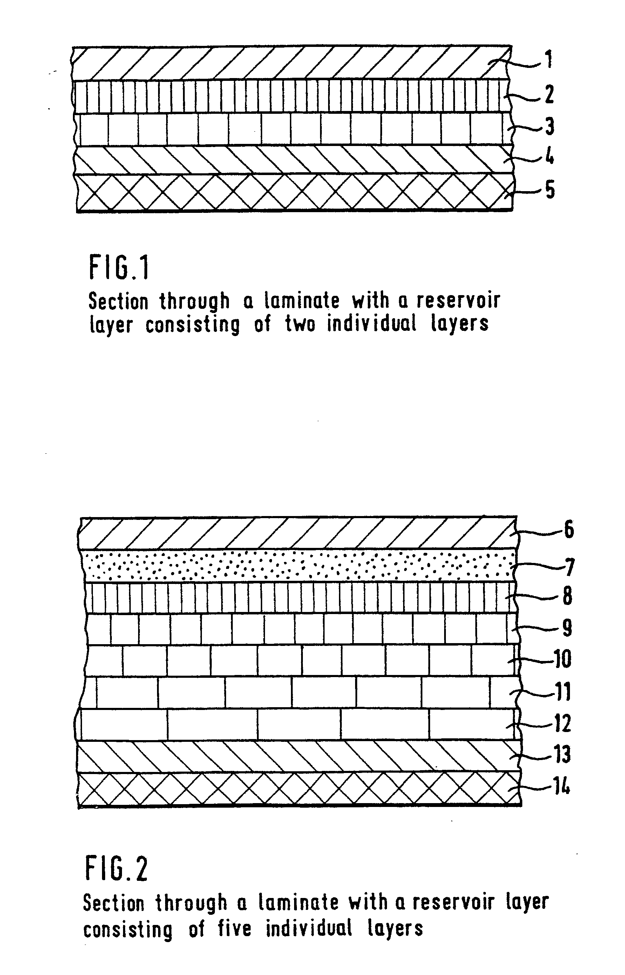

Figure 1 shows a cross section through the embodiment having a two-layer-reservoir. The backing (1) is the most outer layer of the laminate. This layer is a protective layer and the structural base of the pharmaceutical product and substanstially avoids the loss of any component of the individual layers (2) and (3).

The backing layer (1) is followed by the first reservoir layer (2). This layer is immediately below the backing layer and is in close contact with the inner surface of the backing layer. This first reservoir layer is immediately followed by a second reservoir layer. Both reservoir layers (2) and (3) consist of a polymer matrix which in each layer may be equal or different from each other and which both are supersaturated with the active agent contained therein. The content of active agent in both layers is such that it is larger in layer (2) then in layer (3). This is graphically shown in figure (1) by a different hatching.

Immediately after the reservoir layer (3) follows an adhesive layer (4) permeable for the active agent or agents. This layer is to affix the product tensily on the skin. If necessary, it may be loaden also with active agent during the production, the concentration however being smaller or only equal to the saturation concentration.

The adhesive layer (4) is followed by a cover layer which immediately before use is peeled off and removed.

This layer is produced from a material which is not permeable for any of the components of the pharmaceutical product.

Figure 2 shows a cross section of another embodiment according to the present invention with a reservoir built up of five individual layers. Different from Figure 1, there is provided an additional adhesive layer (7) between the backing layer (6) and the upper most reservoir layer (8). Such an additional adhesive layer is preferred and therefore provided in accordance with the present invention if there is no sufficient adhesive power between the upper most layer of the drug reservoir and the backing layer. The reservoir in this embodiment consists of the five layers (8) to (12) which all are supersaturated with the therapeutically active agent. The content of each layer decreases from layer (8) to layer (12) (see difference in hatching). Layer (12) is followed by an adhesive layer (13) permeable to the active agent or agents which layer then is followed by the removable protective layer.

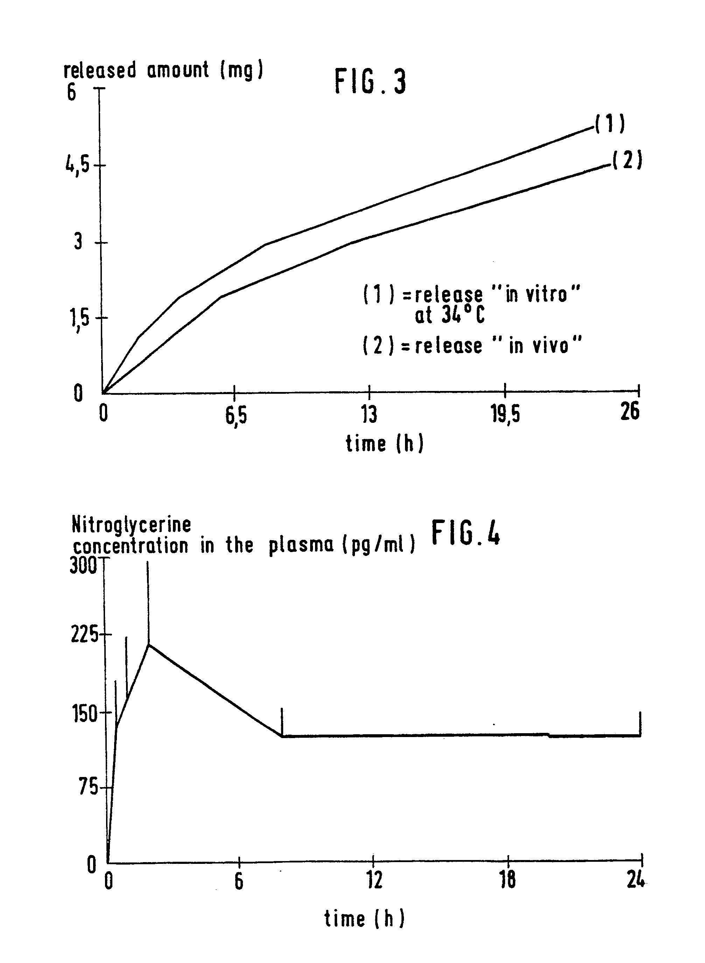

Figures 3 and 4 are further explained in Example 1.

The backing layers (1) or, respectively, (6) may be produced from a flexible or non-flexible material and may have a unilayer or multilayer structure. Materials which may be used for their production are polymers such as polyethylene, polypropylene, polyethyleneterephthalate, polyamide. Furthermore, metal foils such as aluminum foils may be used alone or coated with any of the above polymer materials. Furthermore, the backing layer may also be of textile material if the components of the reservoir layer physically allow such backing material and do not penetrate through textile materials. In a preferred embodiment, the backing layer (1) or, respectively, (6) is of a compact material giving the pharmaceutical product the structural stability and serving as a barrier against the loss of components of the pharmaceutical product according the the present invention. Furthermore, there may be used foils or compact materials coated by aluminum by damping.

The reservoir layers (2) and (3) or, respectively, (8) to (12) consist of a polymer matrix and the therapeutical agent or agents the polymer matrix having such an adhesiveness assuring to keep together the individual reservoir layers. The polymer matrix consists of a base polymer and usual additives. The choice of base polymer depends upon the chemical and physical properties of the used therapeutically active agent or agents. For instance, useful polymers are caoutchouc and caoutchouc-like synthetic homo-, co- or graft polymeres, polyacrylic acid esters and their copolymers, polyurethane and silicon rubbers. All polymers may be used which have been used in the production of pressure sensitive adhesive materials and which are physiologically acceptable.

The kind of additives depends upon the used polymer and upon the therapeutically active agent or agents. There may be plasticizers, agents improving the adhesive power, resorption improving agents, carrier materials, stabilizing agents and fillers. Products which may be used for this purpose and which are physiologically acceptable are known to the expert in the art.

Furthermore, there may be present in the polymer matrix carrier agents for the therapeutically active agent which add to the stabilization of the system and the use of the therapeutically active agent or agents such as lactose in the use of nitroglycerin-lactose-mixtures.

The transdermal therapeutic system according to the present invention may be applied to many therapeutically active agents which are administered to the skin with or without resorption improving agents and which produce a local or systemic activity. Active agents which produce a local effect are, for instance, however without limiting the invention hereto, agents against transpiration, fungicides, bactericides and bacteriostatics.

Therapeutically active agents which produce a systemic activity are, for instance, without however limiting the invention hereto, antibiotics, hormons, antipyretics, antidiabetics, coronar dilatatory agents, heart active glycosides, spasmolytics, antihypertonic, psychopharmaca, antimigraine agents, corticosteroides, analgetics, anticontraceptives, antirheumatics, anticholinergic agents, sympatolytics, sympatomimetics, vasodilatatory agents, anticoagulantives, antiarrhytmetics.

The increase of active agent concentration in the individual layers of the reservoir may cause a decrease in the adhesive power between the outer surface of the reservoir layer and the backing layer which is necessary for the structural stability of the system. In this case, according to the present invention, the structural stability is improved by providing an additional adhesive intermediary layer (7). This layer may be produced from the same material as the polymer matrix without however the resorption improving agents and carrier materials.

The adhesive layer (4) or, respectively, (13) producing the contact to the skin consists of an adhesive material which is physiologically acceptable and which is permeable to the therapeutically active agent or agents in the reservoir layer. Polymer materials useful for this layer may be selected from the list of polymer materials given for the reservoir layer. In order to allow the desired drug release right from the beginning of the use of the pharmaceutical product according to the invention it may be necessary to incorporate into this adhesive layer the active agent or agents during the production of the pharmaceutical product. In this case the active agent concentration is to be lower than, or equal to, the saturation concentration.

The removable or peelable cover layer (5) or, respectively, (14) adjacent to and adhearing to the adhesive layer and which is to be peeled off before use, consists for instance of the same materials as they are used for the backing layer (1) or, respectively, (6), provided however that they are rendered removable from the adhesive layer, for instance by adding a usual silicon coating before application to the adhesive layer. Further materials useful for the production of removable cover layer are for instance polytetrafluoroethylene, paper treated and coated with such material, cellophan, polyvinylchloride or the like. If the pharmaceutical product according to the present invention is cut to for instance medical bandages before application of the cover layer, the then to be applied cover layer cuts may have a projecting part easing the removal of the cover layer from the medical bandage.

Surprisingly, a medical bandage according to the present invention combines all desirable biopharmaceutical and technological properties of a therapeutic system as discussed hereinafter:

1. Control release of active agent

The total of all features of the medical plaster according to the present invention assures a substantially uniform release in active agent for most of the time of administration. If a peak release in the first part of the period of administration is desired, active agent is incorporated into the adhesive layer too.

2. Controllability of the release rate of active agent

The desired rate of release of active agent during the intended time of administration may be controlled in a wide range by the following:

- Composition of the polymer matrixes,
- total of concentration of active agent in the reservoir and, possibly, adhesive layer,
- active agent concentration gradiant over the individual layers of the reservoir layer,
- number of the individual layers of the reservoir layer,
- thickness of the individual layers of the reservoir layer,
- size of the medical plaster,
- kind and amount of carrier agents added to the reservoir layer.

Some or all of these feature may be individually adjusted thereby allowing to meet all desired medical needs.

3. Controllability of the release time

The duration of the therapeutically necessary release rate may be controlled by the chosen proportion between the amount of active agent in the pharmaceutical product and the average release rate.

4. Dosing

Contrary to prior art medical plasters, the dosage per unit of surface area of the adhesive layer is practically not limited in the present medical plaster, obviously due to the particular structure of the reservoir layer as a set of a multitude of individual layers each individual layer being supersaturated with the therapeutically active agent or agents and the particular increase of the concentration of active agent or, respectively, agents from individual layer to individual layer and with increasing distance from the adhesive layer. Furthermore, local irritations on the skin quite often occuring by too high a concentration in active agent, are avoided by the fact that the first individual layer closest to the skin of the treated person is separated from the skin by an adhesive layer which to the most is saturated in active agent or agents, thus avoiding a direct contact of undissolved active agent or agents with the skin.

5. Possibilities to vary the drug releasing surface

Since the pharmaceutical product according to the present invention neither needs lateral walls nor covers nor edge tightening, it may be produced deliberately as large as necessary and in form according to the therapeutical requirements. This is of particular importance where the treatment is started with a minimum dosage, said dosage being slowly increased to the regular dosage or where a treatment is finished with a slowly decreasing dosage.

6. In vitro- in vivo-correlation of drug release

Surprisingly, the pharmaceutical product according to the present invention fulfills the high prerequisites with respect to the in vitro and in vivo drug release. The correlation of in vitro drug release to in vivo drug release is so satisfactory that in vitro test models are most analogous. This allows a secure checking of the reproducability of charges and of bioequivalence.

The structure of the pharmaceutical product according to the present invention is further illustrated hereinafter:

The number of individual layers in the drug reservoir layer is chosen according to the demands. The lowest limit as per definition is 2 individual layers, the upper limit is determined by practical and economical reasons at 12. In a preferred embodiment of the present invention the number of the individual layers is between 2 to 6. The individual layers are of equal or differing thickness, each individual layer having a thickness between from o.oo5 to 5.o mm. Preferably, the thickness of the individual layer is between o.o1 to o.5 mm.

The adhesive layer has a layer thickness of from o.oo5 to 3.o mm, preferably o.o1 to o.5 mm.

The various individual layers of the reservoir layer may be produced from one and the same polymer matrix or the individual layers may be produced from differing polymer matrixes. The amount of therapeutically active agent or agents in the total reservoir layer corresponds to up to the tenfold of the therapeutically desired amount. This therapeutically desired amount is determined by the kind of the active agent or agents, the intended time of the application of the medical bandage and the intended therapeutical field or therapeutical indication for the pharmaceutical product.

The ratio of drug concentration in g per $cm^3$ in the individual layer of the supersaturated reservoir layer adjacent to the adhesive layer to the drug concentration in the individual layer of the supersaturated reservoir layer closest to the cover layer is within the range of 1:1.1 to 1:2o, preferably 1:2 to 1:2o.

The pharmaceutical product according to the present invention is produced applying known technologies in this field. Thus, at first the adhesive layer permeable to the therapeutically active agent or agents is coated to the removable cover layer. Onto this layer are coated the various individual layers of the supersaturated reservoir layer and finally there is coated the final impermeable backing layer onto the last individual layer of the supersaturated reservoir layer. According to the invention there is coated onto the adhesive layer one individual layer of the supersaturated drug reservoir layer and, at least, thereto one further individual layer of the supersaturated reservoir layer wherein the therapeutically active agent or agents are present at a higher concentration in g per $cm^3$ than in the previously coated individual reservoir layer. If desired or necessary, an additional adhesive layer is coated on the last individual reservoir layer before application of the impermeable backing layer. In another embodiment of the present process, the layers may be coated onto each other in reciprocal sequence, i.e. starting with the backing layer, continuing with the individual layer of the reservoir layer having the highest concentration in active agent and continuing with the other individual layers of the reservoir layer with decreasing drug concentrations in each individual layer, then the adhesive layer and finally the removable cover layer.

The adhesive layer, intermediary layer and/or various individual layers of the reservoir layer are produced by laminar distribution of the components of the layers containing additionally a solvent or dispersing agent and removing the solvent or, respectively, dispersing agent to the greatest extent before coating the next layer on to it. Another possibility for producing the various layers consists in converting the components of the layer without solvent or dispersing agent into flat parts from a melt thereof by known methods and to line the various layers on to each other thus forming a laminate. The heat stability of all components at the necessary procedure temperature is a prerequesite for this embodiment of the production of the pharmaceutical product of the present invention.

The following Examples serve to further illustrate the present invention without however limiting the same thereto.

Example 1

A pharmaceutical product according to the present invention having a reservoir layer consisting of three individual layers is produced as follows:

The material for the adhesive layer containing nitroglycerine is produced from

| o.175 kg | of polyisobutylene (mean molecular weight between 9oo,ooo to 1,4oo,ooo. Trade product OPPANOL® B 1oo) |
| o.157 kg | of solid aliphatic hydrocarbon resin (trade product PICCOTAC CBHT) |
| o.157 kg | of hydrogenated rosin resin (trade product ABITOL) |
| o.o1o5 kg | of $^5$ per cent solution of nitroglycerine in a triglycerid of medium-size chain in the ether hydrocarbon group (trade product MIGLYOL® 812), |
| 1.174 kg | of special gasoline 8o - 1oo as solvent |

This product is coated onto the one side of a cover layer having and aluminum layer unilaterally vapour deposited thereon and an abhesive layer at both surfaces such that after evaporation of the solvent a layer of about 2o g per square metre is obtained. Onto this adhesive layer there is coated the first reservoir layer again at a weight per unit of area of about 2o g per square metre.

This reservoir layer is produced by coating a dispersion consisting of

| o.05 kg | of a 1o per cent (weight/weight) nitroglycerine-lactose-distribution, |
| o.153 kg | of polyisobutylene (mean molecular weight of 9oo,ooo to 1,4oo,ooo; trade product OPPANOL B 1oo), |
| o.137 kg | of solid aliphatic hydrocarbon resin (trade product PICCOTAC CBHT), |
| o.137 kg | of hydrogenated rosin resin (trade product ABITOL®) |
| o.o1 kg | of triglyceride as solvent (trade product MIGLYOL 812), |
| 1.148 kg | of special gasoline 8o - 11o as solvent. |

This produce is coated to a separating paper and the dispersion agent and solvent is evaporated thereafter.

In the same way there is produced the bulk material for the second reservoir layer from the following products and coated onto the above first reservoir layer:

| o.6 kg | of a 1o per cent (weight/weight) nitroglycerine-lactose-distribution, |
| o.2 kg | of solid aliphatic hydrocarbon resin (trade product PICCOTAC CBHT), |
| o.2 kg | of hydrogenated rosin resin (trade product ABITOL), |
| o.o25 kg | of triglyceride as solvent (trade product MIGLYOL 812), |
| 1.876 kg | of the special gasoline 8o - 11o as solvent. |

Correspondingly, there is produced the bulk material for the third individual reservoir layer from:

| 2.5 kg | of a 1o per cent (weight/weight) nitroglyerine-lactose-distribution, |
| o.857 kg | of polyisobutylene (mean molecular weight of 9oo,ooo to 1,4oo,ooo; trade product OPPANOL B 1oo), |
| o.77 kg | of solid aliphatic hydrocarbon resin (trade product PICCOTAC CBHT), |
| o.77 kg | of hydrogenated rosin resin (trade product ABITOL), |
| 7.5o7 kg | of the special gasoline 8o - 11o as solvent, |
| o.1 kg | of triglycerid as solvent (trade product MIGLYOL 812). |

For obtaining a total weight per area unit of about 2oo g per square metre, the dispersion is coated onto the separating paper in three consecutive steps. The thus produced third individual layer material is coated onto the second individual reservoir layer.

In a corresponding manner, the additional adhesive intermediary layer is produced with a weight per unit of area of about 2o g per square metre from a mixture of the following components.

| o.179 kg | of polyisobutylene (mean molecular weight 9oo,ooo to 1,4oo,ooo; trade product OPPANOL B 1oo), |
| o.16 kg | of solid aliphatic hydrocarbon resin (trade product PICCOTAC CBHT), |
| o.16 kg | of hydrogenated rosin resin (trade product ABITOL), |
| 1.167 kg | of the special gasoline 8o - 11o as solvent. |

This material is coated onto the third individual reservoir layer.

After finally covering the additional adhesive intermediary layer with an impermeable backing layer, the resulting laminate is divided into the singular medical plasters in accordance with the therapeutic requests.

Stability tests

The stability is determined with cuts of the above laminate measuring 4 by 4 centimetre (= 16 square centimeter). They have been stored in the open atmosphere for 12 weeks at 31°C or 4o°C and 7o per cent relative humidity. The results of these stability tests are summarized in the following table:

## Table I

| Period of storage (weeks) | Nitroglycerine content (%) (mean values ± rel. S.D., n = 3) | |
|---|---|---|
| | 31°C | 4o°C |
| 0 | 100 ± 1.83 | 100 ± 1.83 |
| 2 | 100.8 ± o.57 | 100.3 ± 2.1 |
| 4 | 98.3 ± o.55 | 95.3 ± o.5 |
| 8 | 99.2 ± 3.17 | 94.9 ± o.5 |
| 12 | 99.7 + o.77 | 97.2 + o.58 |

Release of therapeutically active agent

1. in vitro test

A piece of the laminate measuring 16 square centimetres ($cm^2$) and produced in accordance with Example 1, after removal of the cover layer, is dipped into an isotonic sodium chloride solution at 34°C and the amount of released nitroglycerine is determined at predeterminded time intervals by liquid chromatography. The volume of the extraction medium is chosen such that sink conditions are maintained over the total time of the test.

2.1 in vivo test

For each administration three medical plasters, each 16 square centimetre large, produced in accordance with Example 1, are stuck to the chest skin of the test person. After 6, 12 and 26 hours, respectively, one of the medical plasters is pulled off and the nitroglycerine content remaining in the medical plaster is determined chromatographically.

2.2 in vivo test

A 16 square centimetre medical plaster produced according to Example 1 is stuck to the chest skin each of 6 test persons. After 24 hours the medical plaster is pulled off and the nitroglycerine content which remained in the medical plaster is determined chromatographically. The mean value of released nitroglycerine was 5.o ± o.7 mg per 24 hours. Thus, the in vitro test and in vivo test described hereinabove in para. 1 and 2.2 show that there is an excellent in vitro/in vivo-correlation between the amounts of released active agent.

The results are graphically demonstrated in Figure 3. The curves (1) and (2) demonstrating the amount of released active agent show that during the therapeutically intended duration of 24 hours, the nitroglycerine is released from the medical plaster according to the present invention in a controlled and continuous manner, the release rate being almost constant over almost 2o hours.

Bioavailability

Blood samples were taken from the test persons involved in the above tests o.5, 1, 2, 8 and 24 hours after administration and nitroglycerine concentration in the blood plasma was determined by capillary gas chromatography. The results are shown in Figure 4. According thereto, the nitroglycerine concentrations are within the therapeutically active dosage range during the duration of administration.

Example 2

Example 1 was repeated, using a semi-liquid aliphatic hydrocarbon resin in place of the hydrogenated rosin resin, the amounts of the various components being identical otherwise. The coating and sequence of coating is identical with that described in Example 1.

The results of the in vitro test and in vivo test for active agent release are the same as in Example 1. The rate of in vitro release in 24 hours amounted to 3.5 mg.

Example 3

Another pharmaceutical product according to the present invention is produced having a reservoir layer composed of two individual layers:

The nitroglycerine containing adhesive layer bulk material consisted of:

| | |
|---|---|
| 2o g | of polyisobatylene (mean molecular weight 9oo,ooo to 1,4oo,ooo; trade product OPPANOL B 1oo), |
| 18 g | of the solid hydrogenated hydrocarbon resin (trade product PICCOTAC CBHT), |
| 12 g | of the liquid hydrogenated hydrocarbon resin (trade product ADTAC), |
| 1 g | of a 5 per cent solution of nitroglycerine in a triglyceride (trade product MIGLYOL 812), |
| 119 g | of n-hexane as solvent. |

This bulk material is coated to a cover layer which was unilaterally damped with aluminum and on both sides provided with an abhesive lining. The bulk material for the adhesive layer is coated onto the cover layer in such an amount that after evaporation of the solvent a layer weight per unit of area is obtained amounting to about 2o g per square metre.

The first individual layer of the reservoir layer is coated onto the thus obtained adhesive layer at a weight per unit of area of about 2oo g per square metre. The bulk material for this individual layer of the reservoir layer consisted of:

| | |
|---|---|
| 33,8 g | of polyisobutylene (mean molecular weight 9oo,ooo to 1,4oo,ooo; trade product OPPANOL B 1oo), |
| 3o.44 g | of the solid hydrogenated hydrocarbon resin (trade product PICCOTAC CBHT), |
| 2o.3o g | of the liquid hydrogenated hydrocarbon resin (trade product ADTAC), |
| 28.75 g | of a 1o per cent (weight/weight) nitroglycerine lactose distribution, |
| 1.69 g | of a triglyceride (trade product MIGLYOL 812), |
| 179.o g | of n-hexane as dispersion agent. |

Until reaching a total weight per unit of area of about 2oo g per square metre, the above dispersion is coated onto the separation paper in two consecutive steps.

In a corresponding manner, the second reservoir layer having a weight per unit of area of about 1oo g per square metre is produced from the following bulk material:

| | |
|---|---|
| 34.3 g | of polyisobutylene (mean molecular weight 9oo,ooo to 1,4oo,ooo; trade product OPPANOL B 1oo), |
| 3o.9 g | of the solid hydrogenated hydrocarbon resin (trade product PICCOTAC CBHT), |
| 2o.6 g | of the liquid hydrogenated hydrocarbon resin (trade product ADTAC), |
| 87.5 g | of a 1o per cent (weight/weight) nitroglycerine-lactose-distribution, |
| 1.7 g | of a glycerine (trade product MYGLYOL 812), |
| 247.o g | of n-hexane as dispersing agent. |

This individual layer of the reservoir layer is coated onto the first individual layer of the reservoir layer.

In a corresponding manner, the additional adhesive intermediary layer having a weight per unit of area of about 4o g per square metre is produced from the following components:

| | |
|---|---|
| 2o.o g | of polyisobutylene (mean molecular weight 9oo,ooo to 1,4oo,ooo; trade product OPPANOL B 1oo), |
| 18.o g | of the solid hydrogenated hydrocarbon resin (trade product PICCOTAC CBHT), |
| 12.o g | of the liquid hydrogenated hydrocarbon resin (trade product ADTAC), |
| 119.o g | of n-hexane. |

This bulk material is coated onto the second individual layer of the reservoir layer.

After covering the additional adhesive intermediary layer with an impermeable backing layer, the resulting laminate is divided into singular pieces in accordance with the therapeutical demands.

Again, the in vitro release rates and in vivo release rates of nitroglycerine are determined as described in connection with Example 1. The release rate in vitro and in vivo were 3.5 mg or, respectively, 3.o mg within 24 hours. With this product again a continuous and controlled release of nitroglycerine is determined.

Example 4

Another pharmaceutical product (medical plaster) according to the present invention with a double layer reservoir containing BUPRANOLOL (i.e. 1-(tert.butylamino)-3-(6-chloro-3-methyl-phenoxy)-2-propanol) as therapeutically active agent, is produced as follows:

A BUPRANOLOL containing adhesive bulk material is produced from

| | |
|---|---|
| 1.115 kg | of polyisobutylene (mean molecular weight 9oo,ooo to 1,4oo,ooo; trade product OPPANOL B 1oo), |
| 1.338 kg | of solid aromatic hydrocarbon resin (trade product PICCOVAR L 6o), |
| 1.338 kg | of medical white oil (trade product ONDINA OIL G 33), |
| o.o8o kg | of diethyltoluamide, |
| o.13o kg | of a bupranolol/Aerosil * )-mixture 1:1, |
| 5.581 kg | of special gasolin 8o-11o as solvent. |

This product is coated to a separating paper, which has been vapor-coated on one side with aluminum and coated on both sides with an abhesive layer, such that after evaporation of the solvent a layer of about 4o g per square metre is obtained.

Upon the adhesive layer so obtained there is coated the first reservoir layer at a weight of about 7o g per square metre.

This reservoir layer is produced by coating a suspension consisting of

| | |
|---|---|
| 1.722 kg | of polyisobutylene (mean molecular weight 9oo,ooo to 1,4oo,ooo; trade product OPPANOL B 1oo), |
| 2.126 kg | of solid aromatic hydrocarbon resin (trade product PICCOVAR L 6o), |
| 2.126 kg | of medical white oil (trade product ONDINA OIL G 33), |
| o.16o kg | of diethyltoluamide, |
| 1.815 kg | of a Bupranolol/Aerosil-mixture 1:1 |
| 12.554 kg | of special gasolin 8o-11o as solvent |

to a separating paper, evaporating the solvent thereafter, and coating it to the above layer in usual manner.

In the same way a second reservoir layer is produced at a weight about 8o g per square metre from

| | |
|---|---|
| 1.734 kg | of polyisobutylene (mean molecular weight 9oo,ooo to 1,4oo,ooo; trade product OPPANOL B 1oo), |
| 2.o81 kg | of solid aromatic hydrocarbon resin (trade product PICCOVAR L 6o), |
| 2.o81 kg | of medical white oil (trade product ONDINA OIL G 33), |
| o.16o kg | of diethyltoluamide, |
| 1.945 kg | of Bupranolol/Aerosil-mixture 1:1, |
| 12.315 kg | of special gasolin 8o-11o as solvent upon the first reservoir layer. |

In the same way the adhesive layer is produced at a weight of about 2o g per square metre from

| | |
|---|---|
| o.588 kg | of polyisobutylene (mean molecular weight 9oo,ooo to 1,4oo,ooo; trade product OPPANOL B 1oo), |
| o.7o6 kg | of solid aromatic hydrocarbon resin (trade product PICCOVAR L 6o), |
| o.7o6 kg | medical weight oil (trade product ONDINA OIL G 33), |
| 2.944 kg | of special gasolin 8o-11o as solvent |

upon the second reservoir layer.

After covering the adhesive layer with an impermeable cover layer the resulting laminate is cut into single pieces, if desired of varying sizes, as they are needed for the various therapeutic uses.

Release of active agent (in vitro test)

A piece of the laminate measuring 16 square centimetres (cm$^2$) and produced according to the Example 4, after removal of the cover layer, is dipped into an isotonic phosphate buffer solution at 32°C and the amount of released Bupranolol is determined by infrared photometry at predetermined time intervals. The volume of the extraction medium is chosen such that sink conditions are maintained over the total time of the test.

*)Aerosil   is a finely   divided   pure   silicic   acid   product   widely   used   as filler.

| Hours | mg per 16 cm$^2$ |
|---|---|
| 2 | 4.44 |
| 4 | 6.99 |
| 8 | 1o.33 |
| 24 | 19.87 |

## Example 5

A further pharmaceutical product according to the present invention again with a twice layer reservoir containing VERAPAMIL (i.e. 5-(N-(3.4-dimethoxyphenethyl)-N-methylamino)-2-(3.4-dimethoxyphenyl)-2-isopropyl-valeronitrile) as therapeutically active agent, is produced as follows:

The VERAPAMIL containing adhesive bulk material consisting of

| | |
|---|---|
| 1.371 kg | of polyisobutylene (mean molecular weight 9oo,ooo to 1,4oo,ooo; trade product OPPANOL B 1oo), |
| 1.234 kg | of solid aromatic hydrocarbon resin (trade product PICCOVAR L 6o), |
| 1.234 kg | of polyterpene resin (trade product DERCOLYTE S 1o), |
| o.o8o kg | of triglyceride (trade product MIGLYOL 812), |
| o.o8o kg | of VERAPAMIL, |
| 7.685 kg | of special gasolin 8o-11o as first solvent, |
| o.474 kg | of chloroform as second solvent, |

is coated on one side of a protective layer, vapor-coated on one side with aluminum and coated on both sides by abhesive agents, such that after evaporation of the solvents a layer of about 45 g per square metre is produced.

Upon the so obtained adhesive layer there is coated a first reservoir layer at a weight of about 8o g per square metre.

This first reservoir layer is produced by coating a solution consisting of

| | |
|---|---|
| 2.471 kg | of polyisobutylene(mean molecular weight 9oo,ooo to 1,4oo,ooo; trade product OPPANOL B 1oo), |
| 2.224 kg | of solid aromatic hydrocarbon resin (trade product PICCOVAR L 6o), |
| 2.224 kg | of polyterpene resin (trade product DERCOLYTE S 1o), |
| o.16o kg | of triglyceride (trade product MIGLYOL 812), |
| o.92o kg | of VERAPAMIL, |
| 13.851 kg | of special gasolin 8o-11o as first solvent, |
| 5.446 kg | of chloroform as second solvent, |

to a separating paper, evaporating the solvents and applying it to the above adhesive layer in usual manners.

In the same way there is produced a second reservoir layer with a weight of about 85 g per square metre from

| | |
|---|---|
| 2.443 kg | of polyisobutylene (mean molecular weight 9oo,ooo to 1,4oo,ooo; trade product OPPANOL B 1oo), |
| 2.199 kg | of solid aromatic hydrocarbon resin (trade product PICCOVAR L 6o), |
| 2.199 kg | of polyterpene resin (trade product DERCOLYTE S 1o), |
| o.16o kg | of triglyceride (trade product MIGLYOL 812), |
| 1.ooo kg | of VERAPAMIL, |
| 13.695 kg | of special gasolin 8o-11o as first solvent, |
| 5.92o kg | of chloroform as second solvent. |

This product is coated to the first reservoir layer in usual manners.

In the same way there is produced the intermediary adhesive layer having a weight of about 2o g per square metre from

| | |
|---|---|
| o.714 kg | of polyisobutylene (mean molecular weight 9oo,ooo to 1,4oo,ooo; trade product OPPANOL B 1oo), |
| o.643 kg | of solid aromatic hydrocarbon resin (trade product PICCOVAR L 6o), |
| o.643 kg | of polyterpene resin (trade product DERCOLYTE S 1o), |
| 4.oo3 kg | of special gasolin 8o-11o as solvent. |

This product is coated to the second reservoir layer.

After finally covering the intermediary adhesive layer with an impermeable cover layer, the resulting laminate is divided into the singular medical plasters as desired for with the various therapeutic uses.

Release of active agent (in vitro test)

A piece of 25 square centimetres of the laminate produced according to the above Example 5, after removal of the cover layer, is dipped into isotonic phosphate buffer solution at 32°C and the amount of released VERAPAMIL is determined by infrared photometry at predetermined time intervals. The volume of extraction medium is chosen such that sink conditions are maintained over the total time of the test.

| Hours | mg per 25 $cm^2$ |
|-------|------------------|
| 2     | 2.36             |
| 4     | 3.23             |
| 8     | 4.98             |
| 24    | 8.o9             |

## Claims

1. Pharmaceutical product in medical bandage form for the controlled release of a therapeutically active agent to the skin having a reservoir layer of a polymer material permeable to the therapeutically active agent, wherein the therapeutically active agent is soluble and wherein there is a profile of concentration of the therapeutically active agent such that the concentration is higher in the area farther from the surface of the reservoir towards the skin than closer thereto and having an adhesive layer on the side towards the skin, characterised in that the medical bandage consists of an impermeable backing layer, the reservoir layer adjacent to, and in close contact with, said backing layer, the adhesive layer adjacent to, and in close contact with, said reservoir layer and a cover layer covering and adhering to said adhesive layer and removable therefrom for the use of said pharmaceutical product as transdermal therapeutic system, said reservoir layer for the therapeutically active agent
   a) consisting of a multitude of individual polymer matrix layers,
   b) each said individual layer having a concentration of said therapeutically active agent above saturation with the therapeutical active agent both in dissolved and undissolved state, and
   c) the concentration of the therapeutically active agent in said individual layers increasing from individual layer to individual layer with increasing distance from said adhesive layer.

2. The pharmaceutical product as claimed in claim 1, characterized in that it additionally has an adhesive layer between said impermeable backing layer and said supersaturated reservoir layer.

3. The pharmaceutical product as claimed in any of claims 1 and 2, characterized in that said supersaturated reservoir layer consists of 2 to 12, preferably of 2 to 6 individual layers.

4. The pharmaceutical product as claimed in any of claims 1 to 3, characterized in that said individual layers are equal or different in their individual thickness, the thickness of each individual layer being in the range of from 0.005 to 5.0 mm, preferably of from 0.01 to 0.5 mm.

5. The pharmaceutical product as claimed in any of claims 1 to 4, characterized in that said adhesive layer has a thickness in the range of from 0.005 to 3.0 mm, preferably of from 0.01 to 0.5 mm.

6. The pharmaceutical product as claimed in any of claims 1 to 5, characterized in that the polymer matrix comprises one or several usual additives selected from the group consisting of the plasticizers, the adhesive agents, the resorption improving agents, the carrier materials, the stabilizing agents, and the fillers.

7. The pharmaceutical product as claimed in any of claims 1 to 6, characterized in that the individual layers forming the supersaturated reservoir layer are equal or different in polymer matrix material.

8. The pharmaceutical product as claimed in any of claims 1 to 7, characterized in that the amount of therapeutically active agent or agents in the supersaturated reservoir layer is up to the tenfold of the

11

therapeutically necessary amount which is determined by the type of therapeutically active agent or agents, the intended duration of administration and the intended field of pharmaceutical use for the therapeutically active agent.

9. The pharmaceutical product as claimed in any of claims 1 to 8, characterized in that the proportion between the concentration of the therapeutically active agent or agents in g per cm$^3$ in the individual layer of said reservoir layer adjacent to said adhesive layer and the concentration of the therapeutically active agent or agents in the individual layer adjacent to said backing layer or, respectively, said additional adhesive layer, is in the range of from 1:1.1 to 1:20, preferably of from 1:2 to 1:20.

10. Process for the production of a pharmaceutical product as claimed in any of claims 1 to 9, characterized in that there is coated onto a removable cover layer said adhesive layer permeable to the therapeutically active agent or agents to be incorporated into the pharmaceutical product, there then are coated consecutively onto said adhesive layer the various individual layers of said supersaturated reservoir layer, first the individual layer with the lowest concentration in therapeutically active agent or agents and thereafter the individual layers with increasing concentration in therapeutically active agent or agents, if desired, there thereafter is coated the additional adhesive layer and finally said backing layer, or wherein the coating procedure is effected in the contrary sequence starting with said backing layer and finalizing with said removeable cover layer.

11. Process as claimed in claim 10, characterized in that there are used individual coating layer materials for building up said reservoir layer the concentration of the therapeutically active agent or agents in said individual layer materials being such that the proportion between the concentration of the therapeutically agent or agents in g per cm$^3$ in the individual layer of said reservoir layer adjacent to said adhesive layer and the concentration of the therapeutically active agent or agents in the individual layer adjacent to said backing layer or, respectively, said additional adhesive layer is in the range of from 1:1.1 to 1:20, preferably of from 1:2 to 1:20.

12. Process as claimed in claims 10 or 11, characterized in that the material for the adhesive and/or the reservoir layers comprises solvent or, respectively, dispersion agent when being used in the coating step, said solvent or dispersion agent is substantially completely removed from said coating material in each coating step before the next coating step is effected.

13. Process as claimed in any of claims 10 to 12, characterized in that one or several of the various coatings is applied by lining.

14. Process as claimed in any of claims 10 to 13, characterized in that all of the individual layers are produced from a melt of the layer material in each instance.

**Revendications**

1. Produit pharmaceutique sous forme de bandage médical pour la libération contrôlée d'un agent thérapeutiquement actif vers la peau, ayant une couche réservoir d'une matière polymère perméable à l'agent thérapeutiquement actif, de telle sorte que l'agent thérapeutiquement actif soit soluble et qu'il y ait un profil de concentration de l'agent thérapeutiquement actif, de façon à ce que la concentration soit plus élevée dans la zone plus éloignée de la surface du réservoir, en direction de la peau, que plus près de cette surface, et comportant une couche adhésive sur le côté dirigé vers la peau, caractérisé en ce que le bandage médical est constitué par une couche support imperméable, la couche réservoir adjacente à, et en contact étroit avec, ladite couche support, la couche adhésive adjacente à, et en contact étroit avec, ladite couche réservoir, et une couche de couverture recouvrant ladite couche adhésive et adhérant à elle et pouvant être séparée de celle-ci, pour l'utilisation dudit produit pharmaceutique comme système thérapeutique transdermique, ladite couche réservoir pour l'agent actif thérapeutique

    a) étant constituée par une multitude de couches de matrices polymères individuelles,

    b) chacune de ces couches individuelles ayant une concentration en ledit agent actif thérapeutique au-dessus de la saturation avec l'agent actif thérapeutique à la fois à l'état dissous et à l'état non dissous, et

    c) la concentration de l'agent actif thérapeutique dans lesdites couches individuelles augmentant d'une couche individuelle à une couche individuelle lorsque la distance à ladite couche adhésive augmente.

2. Produit pharmaceutique tel que revendiqué dans la revendication 1, caractérisé en ce qu'il comporte en outre une couche adhésive entre ladite couche support imperméable et ladite couche réservoir sursaturée.

3. Produit pharmaceutique tel que revendiqué dans l'une quelconque des revendications 1 et 2, caractérisé en ce que ladite couche réservoir sursaturée est constituée par de 2 à 12 de préférence de 2 à 6 couches individuelles.

4. Produit pharmaceutique tel que revendiqué dans l'une quelconque des revendications 1 à 3, caractérisé en ce que lesdites couches individuelles sont identiques ou différentes dans leur épaisseur individuelle, l'épaisseur de chaque couche individuelle étant dans les limites de 0,005 à 5,0 mm, de préférence de 0,01 à 0,5 mm.

5. Produit pharmaceutique tel que revendiqué dans l'une quelconque des revendications 1 à 4, caractérisé en ce que ladite couche adhésive a une épaisseur dans les limites de 0,005 à 3,0 mm, de préférence de 0,01 à 0,5 mm.

6. Produit pharmaceutique tel que revendiqué dans l'une quelconque des revendications 1 à 5, caractérisé en ce que la matrice polymère comporte un ou plusieurs additifs habituels choisis parmi le groupe constitué par les plastifiants, les agents adhésifs, les agents pour améliorer la résorption, les matières supports, les agents stabilisants, et les charges.

7. Produit pharmaceutique tel que revendiqué dans l'une quelconque des revendications 1 à 6, caractérisé en ce que les couches individuelles formant la couche réservoir sursaturée sont identiques ou différentes dans la matière matrice polymère.

8. Produit pharmaceutique tel que revendiqué dans l'une quelconque des revendications 1 à 7, caractérisé en ce que la quantité d'agent thérapeutiquement actif ou d'agents thérapeutiquement actifs dans la couche réservoir sursaturée va jusqu'à 10 fois la quantité thérapeutiquement nécessaire qui est déterminée par le type d'agent ou d'agents thérapeutiquement actif(s), la durée prévue d'administration et le domaine prévu d'utilisation pharmaceutique pour l'agent thérapeutiquement actif.

9. Produit pharmaceutique tel que revendiqué dans l'une quelconque des revendications 1 à 8, caractérisé en ce que la proportion entre la concentration de l'agent thérapeutiquement actif ou des agents thérapeutiquement actifs en g par cm$^3$ dans la couche individuelle de ladite couche réservoir adjacente à ladite couche adhésive et la concentration de l'agent thérapeutiquement actif ou des agents thérapeutiquement actifs dans la couche individuelle adjacente à ladite couche support ou, respectivement, ladite couche adhésive additionnelle, est dans les limites de 1:1,1 à 1:20, de préférence de 1:2 à 1:20.

10. Procédé pour la production d'un produit pharmaceutique tel que revendiqué dans l'une quelconque des revendications 1 à 9, caractérisé en ce que l'on enduit, sur une couche couverture amovible, ladite couche adhésive perméable à l'agent thérapeutiquement actif, ou aux agents thérapeutiquement actifs, devant être incorporés dans le produit pharmaceutique, l'on enduit ensuite sur ladite couche adhésive les diverses couches individuelles de ladite couche réservoir sursaturée, tout d'abord la couche individuelle avec la plus faible concentration en agent ou agents thérapeutiquement actif(s), et ensuite les couches individuelles avec une concentration croissante d'agent ou d'agents thérapeutiquement actif(s), si on le désire, on applique ensuite la couche adhésive additionnelle et enfin ladite couche support, ou ce procédé étant tel que la procédure d'enduction est effectuée dans un ordre inverse en partant de ladite couche support et en finissant avec ladite couche de couverture amovible.

11. Procédé tel que revendiqué à la revendication 10, caractérisé en ce que l'on utilise des matières pour les couches d'enduction individuelle, pour réaliser ladite couche réservoir, la concentration de l'agent ou des agents thérapeutiquement actif(s) dans lesdites matières de couches individuelles étant telle que la proportion entre la concentration de l'agent ou des agents thérapeutiquement actif(s) en g par cm³ dans la couche individuelle de ladite couche réservoir adjacente à ladite couche adhésive, et la concentration de l'agent ou des agents thérapeutiquement actif(s) dans la couche individuelle adjacente à ladite couche support, ou, respectivement, cette couche adhésive additionnelle, est dans les limites de 1:1,1 à 1:20, de préférence de 1:2 à 1:20.

12. Procédé tel que revendiqué dans les revendications 10 ou 11, caractérisé en ce que la matière pour la couche adhésive et/ou la couche réservoir comporte un solvant ou, respectivement, un agent de dispersion lorsqu'on l'utilise dans l'étape d'enduction, le dissolvant ou ledit agent de dispersion, étant essentiellement enlevé en totalité de ladite matière de revêtement dans chaque étape de revêtement avant que l'étape suivante de revêtement ne soit effectuée.

13. Procédé tel que revendiqué dans l'une quelconque des revendications 10 à 12, caractérisé en ce que l'un ou plusieurs des divers revêtements est appliqué par garnissage.

14. Procédé tel que revendiqué dans l'une quelconque des revendications 10 à 13, caractérisé en ce que toutes les couches individuelles sont produites à partir de la matière fondue de la couche, à chaque fois.

## Patentansprüche

1. Pharmazeutisches Produkt in Pflasterform zur kontrollierten Abgabe von Wirkstoffen an die Haut mit einer Reservoirschicht aus einem Polymermaterial, das für den Wirkstoff durchlässig ist, in der der Wirkstoff löslich ist und in darin solches Wirkstoffkonzentrationsprofil besteht, das die Wirkstoffkonzentration in dem von der Oberfläche zur Haut hin weiter entfernt gelegenen Bereich größer als in dem näheren Bereich ist, und mit einer Haftklebeschicht auf der Seite zur Haut hin, **dadurch gekennzeichnet,** daß das Pflaster besteht aus einer undurchlässigen Deckschicht, einer dazu benachbarten und damit eng verbundenen Reservoirschicht, der dieser Reservoirschicht benachbarten und hiermit eng verbundenen Haftklebeschicht und einer die Haftklebeschicht abdeckende, hieran haftende und zum Gebrauch des pharmazeutischen Produkts als transdermales therapeutisches System wieder entfernbare Schutzschicht, wobei die Reservoirschicht für den therapeutischen Wirkstoff
   a) aus einer Vielzahl Polymermatrixeinzelschichten besteht,
   b) jede dieser Einzelschichten eine Wirkstoffkonzentration über der Sättigung derselben hat, mit Wirkstoff sowohl in gelöstem als auch in ungelöstem Zustand, und
   c) die Wirkstoffkonzentration in den Einzelschichten von Schicht zu Schicht mit steigender Entfernung von der Haftklebeschicht ansteigt.

2. Pharmazeutisches Produkt nach Anspruch 1, **dadurch gekennzeichnet,** daß es zusätzlich eine Haftklebeschicht zwischen der undurchlässigen Deckschicht und der übersättigten Reservoirschicht hat.

3. Pharmazeutisches Produkt nach Anspruch 1 oder 2, **dadurch gekennzeichnet,** daß die übersättigte Reservoirschicht aus 2 bis 12, vorzugsweise 2 bis 6 Einzelschichten besteht.

4. Pharmazeutisches Produkt nach Ansprüchen 1, 2 oder 3, **dadurch gekennzeichnet,** daß die Einzelschichten gleich oder unterschiedlich dick sind, wobei die Einzelschichtdicke im Bereich von 0,005 bis 5,0 mm, vorzugsweise von 0,01 bis 0,5 mm liegt.

5. Pharmazeutisches Produkt nach einem oder mehreren der Ansprüche 1 bis 4, **dadurch gekennzeichnet,** daß die Haftklebeschicht eine Dicke im Bereich von 0,005 bis 3,0 mm, vorzugsweise 0,01 bis 0,5 mm besitzt.

6. Pharmazeutisches Produkt nach einem oder mehreren der Ansprüche 1 bis 5, **dadurch gekennzeichnet,** daß die Polymermatrix übliche Zusätze aus der Gruppe der Weichmacher, Klebrigmacher, Resorptionsvermittler, Carrier, Stabilisatoren, Trägerstoffe und Füllstoffe enthält.

7. Pharmazeutisches Produkt nach einem oder mehreren der Ansprüche 1 bis 6, **dadurch gekennzeichnet,** daß die das übersättigte Reservoir bildenden Einzelschichten aus derselben Polymermatrix oder unterschiedlichen Polymermatrixes besteht.

8. Pharmazeutisches Produkt nach einem oder mehreren der Ansprüche 1 bis 7, **dadurch gekennzeichnet,** daß die Wirkstoffmenge im übersättigten Reservoir bis zum 10-fachen der therapeutisch benötigten Wirkstoffmenge beträgt, wobei diese durch die Art des oder der Wirkstoffe(s), die angestrebte Applikationsdauer und die Indikation des pharmazeutischen Produktes bestimmt ist.

9. Pharmazeutisches Produkt nach einem oder mehreren der Ansprüche 1 bis 8, **dadurch gekennzeichnet,** daß das Verhältnis der Wirkstoffkonzentration (g/cm$^3$) in der an die Haftklebeschicht angrenzenden Schicht des übersättigten Reservoirs zu der in der an die Deckschicht angrenzenden Schicht des übersättigten Reservoirs im Bereich von 1:1,1 bis 1:20, bevorzugt 1:2 bis 1:20, liegt.

10. Verfahren zur Herstellung eines pharmazeutischen Produktes nach den Ansprüchen 1 bis 9, **dadurch gekennzeichnet,** daß auf eine wiederablösbare Schutzschicht eine für den oder die Wirkstoff(e) durchlässige Haftklebeschicht, auf die Haftklebeschicht die verschiedenen Einzelschichten der übersättigten Reservoirschicht aufgetragen werden, wobei zuerst die Schicht mit der niedrigsten Wirkstoffkonzentration und dann die Einzelschichten mit steigenden Wirkstoffkonzentrationen aufgebracht werden, und daß gegebenenfalls vor dem Aufbringen der undurchlässigen Deckschicht eine zusätzliche haftklebende Zwischenschicht aufgebracht wird, oder der Aufbau des Laminates in umgekehrter Reihenfolge erfolgt unter Beginn mit der Deckschicht und zum Schluß der entfernbaren Schutzschicht.

11. Verfahren nach Anspruch 10, **dadurch gekennzeichnet,** daß zum Aufbau der Reservoirschicht Einzelschichten verwendet werden, die Wirkstoffkonzentration in den Einzelschichten derart ist, daß das Verhältnis zwischen der Wirkstoffkonzentration (g/cm$^3$) in der Einzelschicht der Reservoirschicht, die zur Haftklebeschicht benachbart ist, zu der an die Deckschicht bzw. zusätzlichen Haftklebeschicht angrenzenden Schicht im Bereich von 1:1,1 bis 1:20, bevorzugt 1:2 bis 1:20, liegt.

12. Verfahren nach Anspruch 10 oder 11, **dadurch gekennzeichnet,** daß als Haftklebe- und/oder Reservoir-Schichten lösungsmittel- oder dispergiermittelhaltige Massen aufgebracht werden und das Lösungsmittel oder Dispergiermittel vor dem Aufbringen der nächsten Schicht im wesentlichen entfernt wird.

13. Verfahren nach einem oder mehreren der Ansprüche 10 bis 12, **dadurch gekennzeichnet,** daß eine oder mehrere Schichten durch Zukaschieren aufgebracht werden.

14. Verfahren nach einem oder mehreren der Ansprüche 10 bis 13, **dadurch gekennzeichnet,** daß alle Einzelschichten aus der Schmelze hergestellt werden.

# FIG.1

**Section through a laminate with a reservoir layer consisting of two individual layers**

# FIG.2

**Section through a laminate with a reservoir layer consisting of five individual layers**

FIG.3

released amount (mg)

(1) = release "in vitro" at 34°C

(2) = release "in vivo"

time (h)

FIG.4

Nitroglycerine concentration in the plasma (pg/ml)

time (h)